# EUROPÄISCHE PATENTSCHRIFT

(11) **EP 0 391 850 B1**
(45) Veröffentlichungstag und Bekanntmachung des Hinweises auf die Patenterteilung: **28.09.1994**
(21) Anmeldenummer: 90810253.6
(22) Anmeldetag: 29.03.1990
(51) Int. Cl.: C07F 9/38, A61K 31/66

(54) **Ungesättigte Aminodicarbonsäurederivate**
Unsaturated amino-dicarboxylic acid derivatives
Dérivés amino-dicarboxyliques insaturés

(30) Priorität: 07.04.1989 CH 1317/89
(43) Veröffentlichungstag der Anmeldung: 10.10.1990
(73) Patentinhaber: CIBA-GEIGY AG, 4002 Basel (CH)
(72) Erfinder: Allgeier, Hans, Dr., D-7850 Lörrach-Haagen (DE)

(56) Entgegenhaltungen:
- EP-A- 0 233 154
- EP-A- 0 302 826

## Beschreibung

Die Erfindung betrifft ungesättigte Aminodicarbonsäurederivate der Formel I 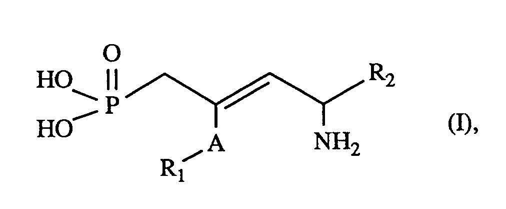
worin A 1,2-Aethylen bedeutet, und R₁ und R₂ für gleiche oder verschiedene C₁-C₄-Alkoxycarbonylgruppen stehen, und ihre Salze, Verfahren zur Herstellung der erfindungsgemässen Verbindungen, diese enthaltende pharmazeutische Präparate und ihre Verwendung als Arzneimittelwirkstoffe.

C₁-C₄-Alkoxycarbonyl ist beispielsweise Methoxycarbonyl, Aethoxycarbonyl, Propoxycarbonyl, Isopropoxycarbonyl oder Butoxycarbonyl.

Die Verbindungen der Formel I können Säureadditionssalze bilden.

Säureadditionssalze von Verbindungen der Formel I sind beispielsweise deren pharmazeutisch verwendbare Salze mit geeigneten Mineralsäuren, wie Halogenwasserstoffsäuren, Schwefelsäure oder Phosphorsäure, z.B. Hydrochloride, Hydrobromide, Sulfate, Hydrogensulfate oder Phosphate, oder Salze mit geeigneten aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate (Cyclamate).

Die Verbindungen der Formel I weisen wertvolle pharmakologische Eigenschaften auf. Insbesondere besitzen sie eine ausgeprägte und selektive antagonistische Wirkung gegenüber N-Methyl-D-asparaginsäure-sensitiven (NMDA-sensitiven) excitatorischen Aminosäurerezeptoren von Warmblütern. Diese kann *in vitro* beispielsweise in der Versuchsanordnung nach G. Fagg und A. Matus, Proc. Nat. Acad. Sci., USA, 81,6876-80 (1984) ermittelt werden. Dabei wird bestimmt, in welchem Ausmass die Bindung von L-³H-Glutaminsäure an NMDA-sensitiven Rezeptoren gehemmt wird. Die NMDA-antagonistischen Eigenschaften der neuen Verbindungen können aber auch *in vivo*, z.B. an der Maus, anhand der Hemmwirkung auf NMDA-induzierte Konvulsionen demonstriert werden.

Die antikonvulsiven Eigenschaften der erfindungsgemässen Verbindungen können beispielsweise an der Maus anhand ihrer ausgeprägten Schutzwirkung gegenüber durch Elektroschock ausgelösten oder audiogen hervorgerufenen Konvulsionen bestimmt werden, wobei man z.B. das etablierte Elektroschock-Mausmodell bzw. die Versuchsanordnung gemäss Chapman et al., Arzneimittel-Forsch. 34,1261(1984) heranziehen kann. Die erfindungsgemässen Verbindungen zeichnen sich dabei, insbesondere im Elektroschock-Mausmodell, durch eine gegenüber strukturverwandten Verbindungen verbesserte Wirkung aus.

Auf Grund dieser Eigenschaften sind die Verbindungen der Formel I und ihre pharmazeutisch verwendbaren Salze vorzüglich geeignet zur Behandlung von pathologischen Zuständen, die auf eine Blockierung von NMDA-sensitiven Rezeptoren ansprechen, beispielsweise von cerebraler Ischämie, ischämischer Erkrankungen des Auges, Muskelspasmen, wie lokale oder generale Spastizität, und insbesondere von Konvulsionen.

Aus EP-A2-233,154 und EP-A2-302,286 waren bereits Verbindungen der Formel I, in denen R₁-A- u.a. Hydroxyniederalkyl oder Niederalkoxyniederalkyl und R₂ verestertes Carboxy bedeutet, denen ähnliche Wirkung zugeschrieben wurde, generisch vorbekannt.

Die Erfindung betrifft namentlich die in den Beispielen genannte Verbindung der Formel I und ihre Salze, insbesondere ihre pharmazeutisch verwendbaren Salze.

Das Verfahren zur Herstellung der erfindungsgemässen Verbindungen ist dadurch gekennzeichnet, dass man
in einer Verbindung der Formel II 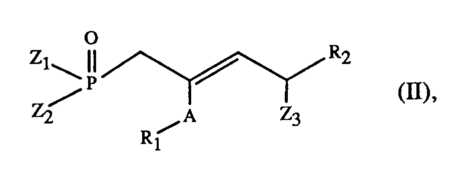
worin Z₁ und Z₂ für gegebenenfalls geschütztes Hydroxy stehen und Z₃ geschütztes Amino bedeutet, Z₃ in Amino und, sofern vorhanden, geschütztes Hydroxy Z₁ und/oder Z₂ in Hydroxy überführt und gewünschtenfalls eine erhaltene Verbindung in eine andere Verbindung der Formel I überführt, ein verfahrensgemäss erhältliches Isomerengemisch in die Komponenten auftrennt und das jeweils bevorzugte Isomere abtrennt und/oder eine verfahrensgemäss erhältliche freie Verbindung in ein Salz oder ein verfahrensgemäss erhältliches Salz in die entsprechende freie Verbindung überführt.

In Ausgangsstoffen der Formel II bedeutet geschütztes Hydroxy Z₁ und/oder Z₂ beispielsweise veräthertes, insbesondere aliphatisch veräthertes Hydroxy, beispielsweise Niederalkoxy, wie Methoxy, Aethoxy oder insbesondere Isopropyloxy, und geschütztes Amino Z₃ beispielsweise acyliertes oder silyliertes Amino.

Acyliertes Amino weist als Acylgruppe beispielsweise von einer organischen Säure, wie einer aliphatischen oder aromatischen Mono- oder Dicarbonsäure oder einem aliphatischen, araliphatischen oder aromatischen Halbester der Kohlensäure abgeleitetes Acyl auf. Acyliertes Amino bedeutet somit beispielsweise Niederalkanoylamino, wie Formyl-, Acetyl- oder Pivaloylamino, gegebenenfalls durch Niederalkyl, Niederalkoxy, Halogen und/oder Nitro substituiertes Benzoylamino, Niederalkoxycarbonylamino, wie Methoxy-, Aethoxy- oder Tertiärbutoxycarbonylamino, gegebenenfalls im Phenylteil durch Niederalkyl, Niederalkoxy, Halogen und/oder Nitro substituiertes Phenylniederalkoxycarbonylamino, wie Benzyloxycarbonylamino, oder durch Niederalkyl, Niederalkoxy, Halogen und/oder Nitro substituiertes Phenoxycarbonylamino.

Silyliertes Amino ist beispielsweise Triniederalkylsilylamino, wie Trimethylsilyl- oder Tributylsilylamino.

Die Freisetzung der geschützten Gruppen aus Verbindungen der Formel II, d.h. von Hydroxy aus geschützten Hydroxygruppen Z₁ und/oder Z₂ und/oder von Amino aus geschützten Aminogruppen Z₃, erfolgt beispielsweise durch Behandeln mit einem sauren Mittel, beispielsweise mit einem Triniederalkylhalogensilan, wie Trimethylbromsilan, Tributylbromsilan oder Trimethyljodsilan, oder, insbesondere zur Herstellung von Verbindungen der Formel I, worin R₁ und R₂ Carboxy bedeuten, mit einer wässrigen Mineralsäure, wie starker, beispielsweise 6-normaler, Salzsäure. Bei der Behandlung mit einem Triniederalkylhalogensilan arbeitet man vorzugsweise in einem inerten Lösungsmittel, wie einem halogenierten aliphatischen Kohlenwasserstoff, z.B. in Dichlormethan oder in zweiter Linie Tri- oder Tetrachlormethan, Trichloräthan oder Tetrachloräthan, beispielsweise im Temperaturbereich von etwa -25° bis etwa +50°C, vorzugsweise von etwa 0° bis 30°C, z.B. bei Raumtemperatur, d.h. bei etwa 15° bis 25°C, vorteilhaft unter weitgehend wasserfreien Bedingungen und unter Inertgas, wie Argon oder Stickstoff. Die Aufarbeitung erfolgt vorteilhaft unter Hinzufügen eines Halogenwasserstoffabfängers, insbesondere einer aliphatischen Epoxyverbindung, wie eines Epoxyniederalkans, z.B. von Propylenoxid in einem Niederalkanol, wie Aethanol. Die Behandlung mit einer wässrigen Mineralsäure erfolgt vorzugsweise unter Erhitzen, z.B. auf etwa 60° bis 120°, vorzugsweise auf Siedetemperatur.

In einer bevorzugten Ausführungsform geht man beispielsweise von Verbindungen der Formel II aus, worin Z₁ und Z₂ Niederalkoxy, z.B. Isopropyloxy, bedeuten und Z₃ Niederalkanoylamino, wie Formylamino, oder Niederalkoxycarbonylamino, wie Tertiärbutoxycarbonylamino, darstellt und behandelt diese in einem aliphatischen Halogenkohlenwasserstoff, wie Dichlormethan, bei etwa 15° bis etwa 25°C mit einem Triniederalkylbromsilan, wie Trimethylbromsilan oder Tributylbromsilan, lässt einige Zeit, z.B. etwa 2 bis 30 Stunden, ausreagieren, fügt dann eine äthanolische Lösung von Propylenoxid hinzu und filtriert das Produkt ab.

Ausgangsstoffe der Formel II werden beispielsweise hergestellt, indem man einen α,β-ungesättigten Aldehyd der Formel IIa 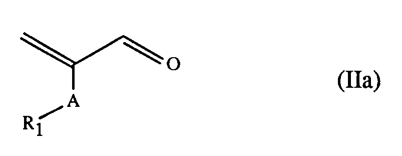
mit einem α-Isocyanessigsäureester der Formel IIb 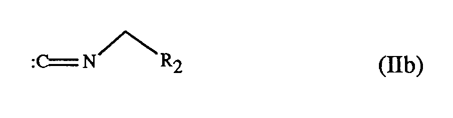
in an sich bekannter Weise, beispielsweise in Gegenwart eines Kupfer- oder Goldkatalysators, z.B. von Kupfer-I-oxid oder insbesondere von Bis(cyclohexylisocyanid)-gold-I-tetrafluoroborat in Gegenwart einer Verbindung der Formel X 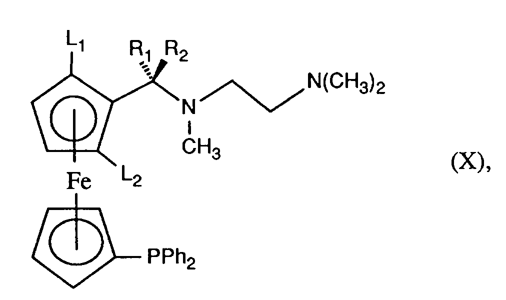
worin einer der Reste L₁ und L₂ sowie PPh₂ Diphenylphosphino und der andere Wasserstoff und einer der Reste R₁ und R₂ Methyl und der andere Wasserstoff bedeutet, zu dem entsprechenden 5-substituierten 2-Oxazolin-4-carbonsäureester der Formel IIc 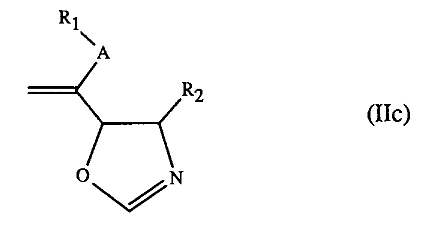
umsetzt, diesen durch Hydrolyse, z.B. in wässrigem Tetrahydrofuran, in den entsprechenden offenkettigen Ester der Formel IId 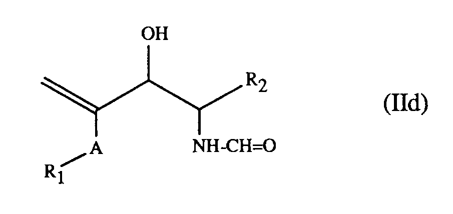
überführt, diesen durch Behandeln mit Thionylbromid in an sich bekannter Weise in den entsprechenden ω-Bromester der Formel IIe 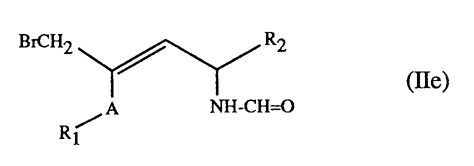
umwandelt und diesen in an sich bekannter Weise mit einem Phosphorigsäuretriester der Formel P(Zₐ)(Z_{b})(Z_{c}), worin Zₐ, Z_{b} und Z_{c} für gleiche oder verschiedene in einer Aetherform geschützte Hydroxygruppen stehen, wie einem Triniederalkylphosphit, z.B. mit Triisopropylphosphit, zur entsprechenden Verbindung der Formel II' 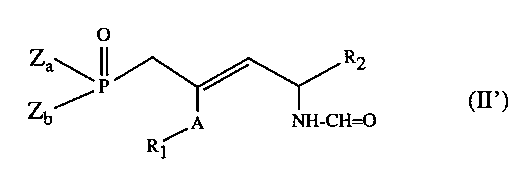
weiterumsetzt.

Verfahrensgemäss erhältliche Salze können in an sich bekannter Weise in die freien Verbindungen umgewandelt werden, z.B. durch Behandeln mit einer Base, wie einem Alkalimetallhydroxid, einem Metallcarbonat oder -hydrogencarbonat, oder Ammoniak, oder einer anderen salzbildenden Base.

Erhaltene Salze können in an sich bekannter Weise in andere Salze überführt werden, Säureadditionssalze z.B. durch Behandeln mit einem geeigneten Metallsalz, wie einem Natrium-, Barium- oder Silbersalz, einer anderen Säure in einem geeigneten Lösungsmittel, in welchem ein sich bildendes anorganisches Salz unlöslich ist und damit aus dem Reaktionsgleichgewicht ausscheidet.

Die Verbindungen der Formel I, einschliesslich ihrer Salze, können auch in Form von Hydraten erhalten werden oder das zur Kristallisation verwendete Lösungsmittel einschliessen.

Infolge der engen Beziehung zwischen den neuen Verbindungen in freier Form und in Form ihrer Salze sind vorstehend und nachfolgend unter den freien Verbindungen und ihren Salzen sinn- und zweckgemäss gegebenenfalls auch die entsprechenden Salze bzw. freien Verbindungen zu verstehen.

Erhaltene Diastereomerengemische und Racematgemische können auf Grund der physikalisch-chemischen Unterschiede der Bestandteile in bekannter Weise in die reinen Diastereomeren bzw. Racemate aufgetrennt werden, beispielsweise durch Chromatographie und/oder fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung des erhaltenen Diastereomerengemisches bzw. Racemates mit einer optisch aktiven Hilfsverbindung, z.B. entsprechend der in Verbindungen der Formel I enthaltenen sauren, basischen oder funktionell abwandelbaren Gruppen mit einer optisch aktiven Säure, Base oder einem optisch aktiven Alkohol, in Gemische diastereomerer Salze bzw. funktioneller Derivate, wie Ester, Trennung derselben in die Diastereomeren, aus denen das jeweils gewünschte Enantiomere in der jeweils üblichen Weise freigesetzt werden kann. Dafür geeignete Basen, Säuren bzw. Alkohole sind beispielsweise optisch aktive Alkaloidbasen, wie Strychnin, Cinchonin oder Brucin, oder D- oder L-(1-Phenyl)äthylamin, 3-Pipecolin, Ephedrin, Amphetamin und ähnliche synthetisch zugängliche Basen, optisch aktive Carbon- oder Sulfonsäuren, wie Chinasäure oder D- oder L-Weinsäure, -Di-o-toluylweinsäure, -Aepfelsäure, -Mandelsäure, oder -Camphersulfonsäure, bzw. optisch aktive Alkohole, wie Borneol oder D- oder L-(1-Phenyl)äthanol.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder insbesondere unter den Reaktionsbedingungen bildet.

Die neuen Ausgangsstoffe, die speziell für die Herstellung der erfindungsgemässen Verbindungen entwickelt wurden, insbesondere die zu den eingangs als bevorzugt gekennzeicheten Verbindungen der Formel I führende Ausgangsstoffauswahl, die Verfahren zu ihrer Herstellung und ihre Verwendung als Zwischenprodukte bilden ebenfalls einen Gegenstand der Erfindung.

Die neuen Verbindungen der Formel I können z.B. in Form pharmazeutischer Präparate Verwendung finden, welche eine therapeutisch wirksame Menge der Aktivsubstanz, gegebenenfalls zusammen mit anorganischen oder organischen, festen oder flüssigen, pharmazeutisch verwendbaren Trägerstoffen enthalten, die sich zur enteralen, z.B. oralen, oder parenteralen Verabreichung eignen. So verwendet man Tabletten oder Gelatinekapseln, welche den Wirkstoff zusammen mit Verdünnungsmitteln, z.B. Lactose, Dextrose, Saccharose, Mannit, Sorbit, Cellulose und/oder Schmiermitteln, z.B. Kieselerde, Talk, Stearinsäure oder Salzen davon, wie Magnesium- oder Calciumstearat, und/oder Polyäthylenglykol, aufweisen. Tabletten können ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, z.B. Natriumalginat, und/oder Brausemischungen, oder Absorptionsmittel, Farbstoffe, Geschmacksstoffe und Süssmittel aufweisen. Ferner kann man die neuen Verbindungen der Formel I in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservierungs-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier-, Lösungs- oder Lyophilisierungsverfahren hergestellt und enthalten von etwa 0,1% bis 100%, insbesondere von etwa 1% bis etwa 50%, Lyophilisate bis etwa 100% des Aktivstoffes.

Die Erfindung betrifft ebenfalls die Verwendung der Verbindungen der Formel I, vorzugsweise in Form von pharmazeutischen Präparaten. Die Dosierung kann von verschiedenen Faktoren, wie Applikationsweise, Spezies, Alter und/oder individuellem Zustand abhängen. Die täglich zu verabreichenden Dosen liegen bei oraler Applikation zwischen etwa 0,25 und etwa 10 mg/kg und für Warmblüter mit einem Körpergewicht von etwa 70 kg vorzugsweise zwischen etwa 20 mg und etwa 500 mg.

Die nachfolgen Beispiele dienen zur Illustration der Erfindung; Temperaturen sind in Celsiusgraden, Drucke in mbar angegeben.

Beispiel 1: 4,3 g (9,9 mMol) E-2-Formylamino-4-diisopropylphosphonomethyl-hept-3-en- 1,7-disäurediäthylester werden in 14 ml Dichlormethan gelöst und bei Raumtemperatur tropfenweise mit 5,2 ml (40 mMol) Trimethylbromsilan versetzt. Man lässt 20 Stunden bei Raumtemperatur stehen, tropft 14 ml Aethanol hinzu, lässt nochmals 19 Stunden stehen, dampft am Rotationsverdampfer ein, nimmt in 14 ml Aethanol auf und versetzt mit einem Gemisch von 14 ml Propylenoxid und 14 ml Aethanol. Es bildet sich eine Suspension, die noch 90 Minuten gerührt und dann abgesaugt wird. Man erhalt E-2-Amino-4-phosphonomethyl-hept-3-en-1,7-disäurediäthylester vom Smp. 218-20° (Zers.)

Das Ausgangsmaterial kann z.B. folgendermassen hergestellt werden:
14,4g (100mMol) 5-Oxopentansäureäthylester, 9,2g (112,6 mMol) Dimethylammoniumchlorid und 10,8 ml (117 mMol) 37%-ige Formaldehydlösung werden unter Rühren 1 Stunde auf 100° erhitzt. Man lässt abkühlen und extrahiert 3-mal mit je 30 ml Diäthyläther. Die organischen Phasen werden vereinigt, mit gesättigter Kochsalzlösung gewaschen, über Magnesiumsulfat getrocknet und zur Trockne eingedampft. Man erhält 4-Formylpent-4-ensäureäthylester als farbloses Oel, das ohne weitere Reinigung weiterumgesetzt werden kann.

14,9g (95 mMol) 4-Formylpent-4-ensäureäthylester und 10,4g (95 mMol) Isocyanessigsäureäthylester werden zu einer Suspension von Kupfer-I-oxid in 50 ml Benzol zugetropft. Nach Abklingen der exothermen Reaktion lässt man noch 45 Minuten bei Raumtemperatur nachrühren, filtriert über Hyflo® und dampft zur Trockne ein. Der Rückstand wird in 75 ml Tetrahydrofuran aufgenommen, mit 25 ml Wasser versetzt und unter Rühren 4 Stunden zum Rückfluss erhitzt. Man dampft zur Trockne ein und chromatographiert an Silicagel mit Toluol/Isopropanol (9:1) als Elutionsmittel. Man erhält 2-Formylamino-3-hydroxy-4-methylen-heptan-1,7-disäurediäthylester als bräunliches Oel.

10,3 g (35,9 mMol) 2-Formylamino-3-hydroxy-4-methylen-heptan-1,7-disäurediäthylester werden in 100 ml Dichlormethan gelöst und bei Raumtemperatur tropfenweise mit 3,34 ml (43,1 mMol) Thionylbromid versetzt. Nach 1 Stunde gibt man 10 ml Wasser hinzu und lässt 10 Minuten intensiv rühren. Die organische Phase wird abgetrennt, nacheinander mit Wasser, gesättigter Kaliumhydrogencarbonatlösung und nochmals mit Wasser gewaschen, über Magnesiumsulfat getrocknet, filtriert und eingedampft. Man erhält 2-Formylamino-4-bromomethyl-hept-3-en-1,7-disäurediäthylester als bräunliches Oel.

8,7 g (25 mMol) 2-Formylamino-4-bromomethyl-hept-3-en-1,7-disäurediäthylester und 21 ml (75 mMol) Triisopropylphosphit (90%-ig) werden auf 80° bis 90°C erwärmt und unter einem Druck von etwa 100 mbar 19 Stunden gerührt. Das überschüssige Triisopropylphosphit wird abdestilliert und der Eindampfrückstand an 150g Silicagel mit zunächst Essigsäureäthylester und dann Essigsäureäthylester/Aethanol (9:1) als Elutionsmittel. Man erhält E-2-Formylamino-4-diisopropylphosphonomethyl-hept-3-en-1,7-disäurediäthylester als gelbliches Oel.

Beispiel 2: Tabletten, enthaltend je 50 mg E-2-Amino-4-phosphonomethyl-hept-3-en-1,7-disäurediäthylester oder ein Salz, z.B. das Natriumsalz, davon können wie folgt hergestellt werden:

| Zusammensetzung (10000 Tabletten) | |
|---|---|
| Wirkstoff | 500,0g |
| Lactose | 500,0g |
| Kartoffelstärke | 352,0g |
| Gelatine | 8,0g |
| Talk | 60,0g |
| Magnesiumstearat | 10,0g |
| Siliciumdioxid (hochdispers) | 20,0g |
| Aethanol | q.s. |

Der Wirkstoff wird mit der Lactose und 292 g Kartoffelstärke vermischt, die Mischung mit einer äthanolischen Lösung der Gelatine befeuchtet und durch ein Sieb granuliert. Nach dem Trocknen mischt man den Rest der Kartoffelstärke, das Magnesiumstearat, das Talk und das Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 3: Lacktabletten, enthaltend je 100 mg E-2-Amino-4-phosphonomethyl-hept-3-en-1,7-disäurediäthylester oder ein Salz, z.B. das Natriumsalz, davon können wie folgt hergestellt werden:

| Zusammensetzung (für 1000 Lacktabletten) | |
|---|---|
| Wirkstoff | 100,0 g |
| Lactose | 100,0 g |
| Maisstarke | 70,0 g |
| Talk | 8,5g |
| Calciumstearat | 1,5 g |
| Hydroxypropylmethylcellulose | 2,36 g |
| Schellack | 0,64 g |
| Wasser | q.s. |
| Methylenchlorid | q.s. |

Der Wirkstoff, die Lactose und 40 g der Maisstarke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat zugegeben und mit dem Granulat vermischt. Das Gemisch wird zu Tabletten (Gewicht: 280 mg) verpresst und diese mit einer Lösung der Hydroxypropylmethylcellulose und des Schellacks in Methylenchlorid lackiert; Endgewicht der Lacktablette: 283 mg.

Beispiel 4: Gelatinesteckkapseln, enthaltend 100 mg Wirkstoff, z.B. E-2-Amino-4-phosphonomethyl-hept-3-en-1,7-disäurediäthylester oder ein Salz, z.B. das Natriumsalz, davon, können z.B. folgendermassen hergestellt werden:

| Zusammensetzung (für 1000 Kapseln) | |
|---|---|
| Wirkstoff | 100,0 g |
| Lactose | 250,0 g |
| mikrokristalline Zellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird zunächst die Lactose durch ein Sieb mit einer Maschenweite von 0,6 mm und dann die mikrokristalline Zellulose durch ein Sieb mit einer Maschenweite von 0,9 mm hinzugesiebt. Daraufhin wird erneut 10 Minuten innig gemischt. Zuletzt wird das Magnesiumstearat durch ein Sieb mit einer Maschenweite von 0,8 mm hinzugesiebt. Nach 3-minütigem weiteren Mischen werden je 390 mg der erhaltenen Formulierung in Gelatinesteckkapseln der Grösse 0 abgefüllt.

Beispiel 5: Eine 0,2%-ige Injektions- oder Infusionslösung von E-2-Amino-4-phosphonomethyl-hept-3-en-1,7-disäurediäthylester oder eines Salzes, z.B. des Natriumsalzes, davon, kann beispielsweise folgendermassen hergestellt werden:

| Zusammensetzung (für 1000 Ampullen) | |
|---|---|
| Wirkstoff | 5,0 g |
| Natriumchlorid | 22,5 g |
| Phosphatpuffer pH=7.4 | 300,0 g |
| entmineralisiertes Wasser | ad 2500,0 ml |

Der Wirkstoff und das Natriumchlorid werden in 1000 ml Wasser gelöst und durch ein Mikrofilter filtriert. Man versetzt mit der Pufferlösung und füllt mit Wasser auf 2500 ml auf. Zur Herstellung von Dosiseinheitsformen werden je 1,0 oder 2,5 ml in Glasampullen abgefüllt, die dann je 2,0 bzw. 5,0 mg Wirkstoff enthalten.

## Patentansprüche (Patentansprüche für folgende(n) Vertragsstaat(en): AT, BE, CH, DK, FR, GB, IT, LI, LU, NL, SE)

1. Ungesättigte Aminodicarbonsäurederivate der Formel I 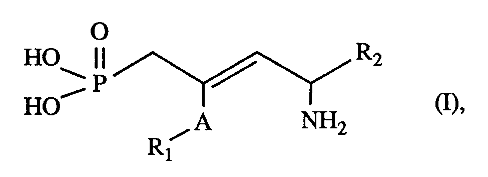 worin A 1,2-Aethylen bedeutet, und R₁ und R₂ für gleiche oder verschiedene C₁-C₄-Alkoxycarbonylgruppen stehen, und ihre Salze.

2. E-2-Amino-4-phosphonomethyl-hept-3-en-1,7-disäurediäthylester oder ein Salz davon.

3. Eine Verbindung gemäss Anspruch 1 zur Anwendung in einem Verfahren zur therapeutischen Behandlung des menschlichen oder tierischen Körpers.

4. Die Verbindung gemäss Anspruch 2 zur Anwendung in einem Verfahren zur therapeutischen Behandlung des menschlichen oder tierischen Körpers.

5. Pharmazeutische Präparate als pharmazeutischen Wirkstoff enthaltend eine Verbindung gemäss einem der Ansprüche 1 und 3 in freier Form oder pharmazeutisch verwendbarer Salzform.

6. Pharmazeutische Präparate als pharmazeutischen Wirkstoff enthaltend eine Verbindung gemäss einem der Ansprüche 2 und 4 in freier Form oder pharmazeutisch verwendbarer Salzform.

7. Verfahren zur Herstellung von Verbindungen der Formel I gemäss Anspruch 1, dadurch gekennzeichnet, dass man
in einer Verbindung der Formel II 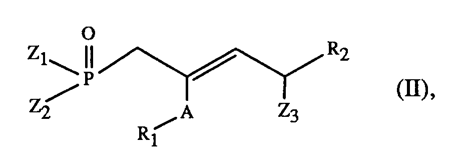 worin Z₁ und Z₂ für gegebenenfalls geschütztes Hydroxy stehen und Z₃ geschütztes Amino bedeutet, Z₃ in Amino und, sofern vorhanden, geschütztes Hydroxy Z₁ und/oder Z₂ in Hydroxy überführt und gewünschtenfalls eine erhaltene Verbindung in eine andere Verbindung der Formel I überführt, ein verfahrensgemäss erhältliches Isomerengemisch in die Komponenten auftrennt und das jeweils bevorzugte Isomere abtrennt und/oder eine verfahrensgemäss erhältliche freie Verbindung in ein Salz oder ein verfahrensgemäss erhältliches Salz in die entsprechende freie Verbindung überführt.

8. Verwendung einer Verbindung gemäß einem der Ansprüche 1 bis 2 zur Herstellung eines pharmazeutischen Präparates.

9. Verwendung einer Verbindung gemäß einem der Ansprüche 1 bis 2 zur Herstellung eines Arzneimittels für die Behandlung pathologischer Zustände, die auf eine Blockierung von NMDA-sensitiven Rezeptoren ansprechen.

## Patentansprüche (Patentansprüche für folgende(n) Vertragsstaat(en): ES, GR)

1. Verfahren zur Herstellung von ungesättigten Aminodicarbonsäurederivaten der Formel I 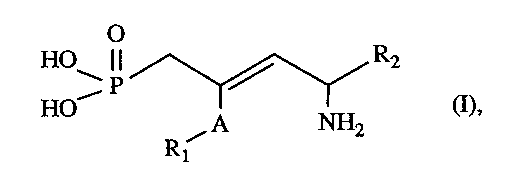 worin A 1,2-Aethylen bedeutet, und R₁ und R₂ für gleiche oder verschiedene C₁-C₄-Alkoxycarbonylgruppen stehen, und ihrer Salze, dadurch gekennzeichnet, dass man
in einer Verbindung der Formel II 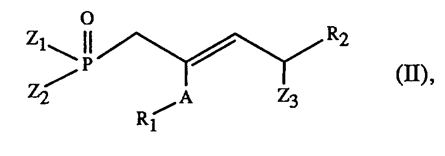 worin Z₁ und Z₂ für gegebenenfalls geschütztes Hydroxy stehen und Z₃ geschütztes Amino bedeutet, Z₃ in Amino und, sofern vorhanden, geschütztes Hydroxy Z₁ und/oder Z₂ in Hydroxy überführt und gewünschtenfalls eine erhaltene Verbindung in eine andere Verbindung der Formel I überführt, ein verfahrensgemäss erhältliches Isomerengemisch in die Komponenten auftrennt und das jeweils bevorzugte Isomere abtrennt und/oder eine verfahrensgemäss erhältliche freie Verbindung in ein Salz oder ein verfahrensgemäss erhältliches Salz in die entsprechende freie Verbindung überführt.

2. Verfahren gemäss Anspruch 1 zur Herstellung von E-2-Amino-4-phosphonomethyl-hept-3-en-1,7-disäurediäthylester oder eines Salzes davon.

3. Verfahren zur Herstellung von pharmazeutischen Präparaten, dadurch gekennzeichnet, dass man eine Verbindung gemäss einem der Ansprüche 1 und 2 mit einem oder mehreren pharmazeitschen Hilfsstoff(en) vermischt.

## Claims (Claims for the following Contracting State(s): AT, BE, CH, DK, FR, GB, IT, LI, LU, NL, SE)

1. An unsaturated aminodicarboxylic acid derivative of formula I 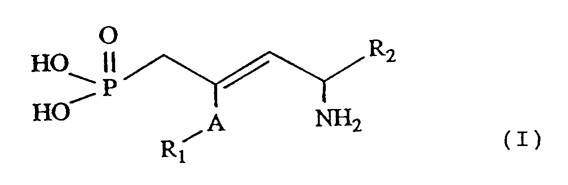 wherein A is 1,2-ethylene, and R₁ and R₂ are the same or different C₁-C₄alkoxycarbonyl groups, or a salt thereof.

2. E-2-Amino-4-phosphonomethylhept-3-ene-1,7-diacid diethyl ester or a salt thereof.

3. A compound according to claim 1 for use in a method for the therapeutic treatment of the human or animal body.

4. A compound according to claim 2 for use in a method for the therapeutic treatment of the human or animal body.

5. A pharmaceutical composition comprising as pharmaceutical active ingredient a compound according to either claim 1 or claim 3 in free form or in the form of a pharmaceutically acceptable salt.

6. A pharmaceutical composition comprising as pharmaceutical active ingredient a compound according to either claim 2 or claim 4 in free form or in the form of a pharmaceutically acceptable salt.

7. A process for the preparation of a compound of formula I according to claim 1 which comprises converting, in a compound of formula II 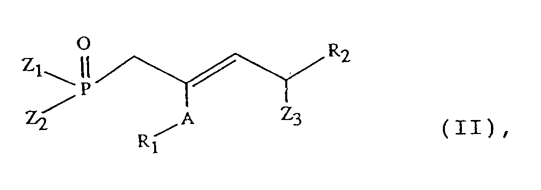 wherein Z₁ and Z₂ are hydroxy or protected hydroxy and Z₃ is protected amino, Z₃ into amino and, if present, protected hydroxy Z₁ and/or Z₂ into hydroxy and, if desired, converting a resulting compound into another compound of formula I, resolving a mixture of isomers obtainable in accordance with the process into the individual components and separating the preferred isomer in each case, and/or converting a free compound obtainable in accordance with the process into a salt or a salt obtainable in accordance with the process into the corresponding free compound.

8. The use of a compound according to any one of claims 1 to 2, for the preparation of a pharmaceutical composition.

9. The use of a compound according to any one of claims 1 to 2, for the preparation of a medicament for the treatment of pathological conditions that respond to a blocking of NMDA-sensitive receptors.

## Claims (Claims for the following Contracting State(s): ES, GR)

1. A process for the preparation of an unsaturated aminodicarboxylic acid derivative of formula I 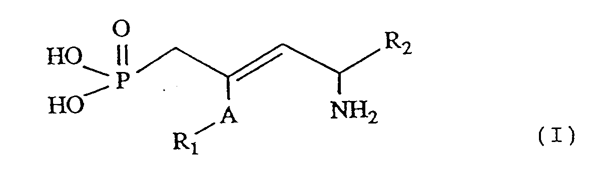 wherein A is 1,2-ethylene, and R₁ and R₂ are the same or different C₁-C₄alkoxycarbonyl groups, or a salt thereof, which comprises converting, in a compound of formula II 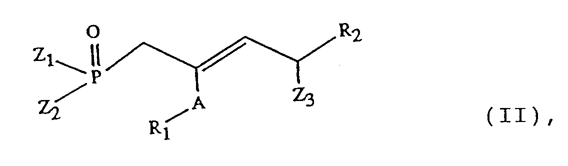 wherein Z₁ and Z₂ are hydroxy or protected hydroxy and Z₃ is protected amino, Z₃ into amino and, if present, protected hydroxy Z₁ and/or Z₂ into hydroxy and, if desired, converting a resulting compound into another compound of formula I, resolving a mixture of isomers obtainable in accordance with the process into the individual components and separating the preferred isomer in each case, and/or converting a free compound obtainable in accordance with the process into a salt or a salt obtainable in accordance with the process into the corresponding free compound.

2. A process according to claim 1 for the preparation of E-2-amino-4-phosphonomethylhept-3-ene-1,7-diacid diethyl ester or a salt thereof.

3. A process for the preparation of a pharmaceutical composition which comprises mixing a compound according to either claim 1 or claim 2 with one or more pharmaceutical excipients.

## Revendications (Revendications pour l'(les) Etat(s) contractant(s) suivant(s): AT, BE, CH, DK, FR, GB, IT, LI, LU, NL, SE)

1. Dérivés d'acides amino-dicarboxyliques insaturés de formule I 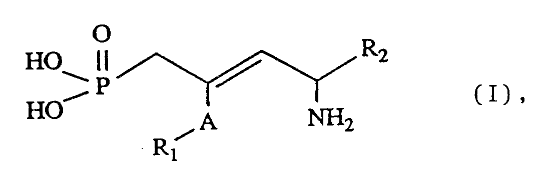 dans laquelle A représente le 1,2-éthylène et R₁ et R₂ représentent des groupes alcoxycarbonyles C₁-C₄ identiques ou différents, et leurs sels.

2. E-2-amino-4-phosphonométhyl-hept-3-èn-1,7-dicarboxylate de diéthyle ou un de ses sels.

3. Composé selon la revendication 1 pour l'utilisation dans un procédé de traitement thérapeutique du corps humain ou animal.

4. Composé selon la revendication 2 pour l'utilisation dans un procédé de traitement thérapeutique du corps humain ou animal.

5. Préparations pharmaceutiques contenant comme principe actif pharmaceutique un composé selon l'une des revendications 1 et 3 sous forme libre ou sous forme de sel pharmaceutiquement utilisable.

6. Préparations pharmaceutiques contenant comme principe actif pharmaceutique un composé selon l'une des revendications 2 et 4 sous forme libre ou sous forme de sel pharmaceutiquement utilisable.

7. Procédé de préparation de composés de formule I selon la revendication 1, caractérisé en ce que
dans un composé de formule II 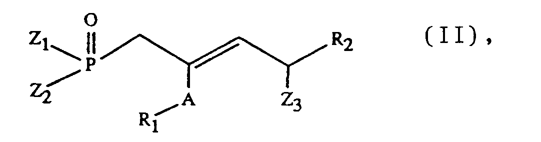 dans lequel Z₁ et Z₂ représentent un hydroxy protégé le cas échéant et Z₃ un amino protégé, on transforme Z₃ en amino et, en cas de présence, on transforme un hydroxy Z₁ et/ou Z₂ protégé en hydroxy et si c'est souhaité, on transforme un composé obtenu en un autre composé de formule I, on fractionne en ses composants un mélange d'isomères qu'on peut obtenir selon le procédé, et on isole l'isomère préféré dans chaque cas et/ou on transforme un composé libre, qu'on peut obtenir selon le procédé, en un sel, ou on transforme un sel qu'on peut obtenir selon le procédé en le composé libre correspondant.

8. Utilisation d'un composé selon l'une des revendications 1 à 2 pour produire une préparation pharmaceutique.

9. Utilisation d'un composé selon l'une des revendications 1 à 2 pour produire un médicament destiné au traitement d'états pathologiques qui correspondent à un blocage des récepteurs NMDA-sensibles.

## Revendications (Revendications pour l'(les) Etat(s) contractant(s) suivant(s): ES, GR)

1. Procédé de préparation de dérivés d'acides amino-dicarboxyliques insaturés de formule I 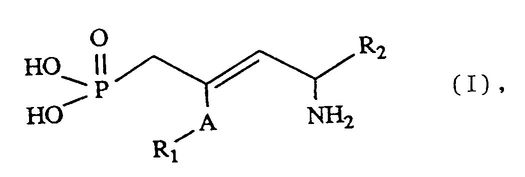 dans laquelle A représente le 1,2-éthylène et R₁ et R₂ représentent des groupes alcoxycarbonyles C₁-C₄ identiques ou différents, et leurs sels, caractérisé en ce que
dans un composé de formule II 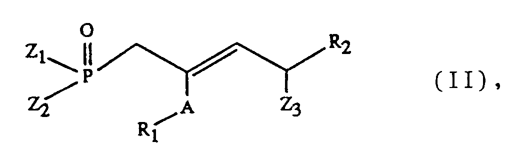 dans lequel Z₁ et Z₂ représentent un hydroxy protégé le cas échéant et Z₃ un amino protégé, on transforme Z₃ en amino et, en cas de présence, on transforme un hydroxy Z₁ et/ou Z₂ protégé en hydroxy et, si c'est souhaité, on transforme un composé obtenu en un autre composé de formule I, on fractionne en ses composants un mélange d'isomères qu'on peut obtenir selon le procédé, et on isole l'isomère préféré dans chaque cas et/ou on transforme un composé libre, qu'on peut obtenir selon le procédé, en un sel, ou on transforme un sel qu'on peut obtenir selon le procédé en le composé libre correspondant.

2. Procédé de préparation selon la revendication 1 de E-2-amino-4-phosphonométhyl-hept-3-èn-1,7-dicarboxylate de diéthyle ou un de ses sels.

3. Procédé de production de préparations pharmaceutiques, caractérisé en ce qu'on mélange un composé selon l'une des revendications 1 et 2 avec une ou plusieurs substances auxiliaires pharmaceutiques.
